(19) Europäisches Patentamt
European Patent Office
Office européen des brevets

(11) Publication number: 0 113 570 B1

(12) EUROPEAN PATENT SPECIFICATION

(45) Date of publication of patent specification: 25.02.87

(51) Int. Cl.[4]: C 07 D 233/88, C 07 D 233/60, C 07 D 401/04, C 07 D 401/06, A 61 K 31/415

(21) Application number: 83307692.0

(22) Date of filing: 16.12.83

(54) 5-(Amino or substituted amino) imidazoles.

(30) Priority: 20.12.82 US 450848

(43) Date of publication of application: 18.07.84 Bulletin 84/29

(45) Publication of the grant of the patent: 25.02.87 Bulletin 87/09

(84) Designated Contracting States: AT BE CH DE FR GB IT LI LU NL SE

(56) References cited:
EP-A-0 028 834
US-A-4 265 900

CHEMICAL ABSTRACTS, vol. 96, no. 9, March 1, 1982, Columbus, Ohio, USA, SUMITOMO CHEMICAL CO. "1-Triarylmethylimidazoles", page 613, column 2, abstract no. 68990k

CHEMICAL ABSTRACTS, vol. 96, no. 17, April 26, 1982, Columbus, Ohio, USA, TH. KAUFFMANN et al. "Heterocyclopolyaromatics XII", page 757, column 2, abstract no. 142 826e

(73) Proprietor: MERCK & CO. INC.
126, East Lincoln Avenue P.O. Box 2000
Rahway New Jersey 07065 (US)

(72) Inventor: Chabala, John C.
560 Sherwood Parkway
Westfield New Jersey 07090 (US)
Inventor: Fisher, Michael H.
R.D. 1, Old York Road
Ringoes New Jersey 08551 (US)
Inventor: Patchett, Arthur A.
1090 Minisink Way
Westfield New Jersey 07090 (US)

(74) Representative: Crampton, Keith John Allen et al
D YOUNG & CO 10 Staple Inn
London WC1V 7RD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 113 570 B1

Courier Press, Leamington Spa, England.

(56) References cited:

**CHEMICAL ABSTRACTS, vol. 94, no. 13, March 30, 1981, Columbus, Ohio, USA, PFIZER CORP., "Imidazole derivatives", page 747, column 1, abstract no. 103363r**

**CHEMICAL ABSTRACTS, vol. 97, no. 25, December 20, 1982, Columbus, Ohio, USA, SANKYO KAGAKU K.K. "8-Quinolinesulfonyl derivatives", page 855, column 2, abstract no. 216195t**

**Description**

This invention relates to anticoccidial compounds and their preparation.

Coccidiosis is a widespred poultry disease produced by infections of protozoa of the genus *Eimeria,* which causes severe pathology in the intestines and caeca of poultry. Some of the most significant of these species are *E. tenella, E. acervulina, E. necatrix, E. brunetti, E. maxima, E. mitis, E. mivati, E. hagini* and *E. praecox.* The disease is generally spread by the birds picking up the infectious organism in droppings on contaminated litter or ground or by way of food or drinking water. The disease is manifested by haemorrhage, accumulation of blood in the caeca, passage of blood to the droppings, weakness and digestive disturbances. The disease often terminates in death but the market value of fowl that survive severe infections is substantially reduced as a result. Coccidiosis is therefore a disease of great economic importance and extensive work has been done to find new and improved methods for controlling and treating coccidial infections in poultry.

This invention is based on the discovery that certain novel 5-amino and substituted amino imidazoles and certain of their substituted derivatives have a surprisingly and unexpectedly high degree of activity against coccidiosis of poultry.

The present invention provides a compound having the formula:

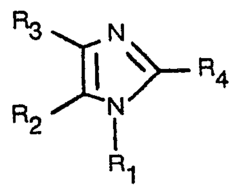

in which $R_1$ is (a) mono-substituted phenyl or mono-substituted phen $C_{1-3}$ alkyl where the substituent is trifluoromethyl, $C_{2-3}$ alkanoyl, nitro, carboxy, alkoxycarbonyl, acetamido, $C_{1-3}$ alkylthio, $C_{1-3}$ alkylsulfinyl, $C_{1-3}$ alkylsulfonyl or

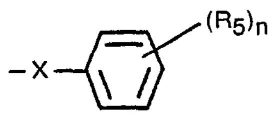

where n is from 0 to 5, $R_5$ is as defined below and X is O, S, SO, $SO_2$, $CH_2$, CO, CHOH, CHCN or $C{=}NR_6$ where $R_6$ is hydrogen, $C_{1-3}$ alkyl, hydroxy, $C_{1-3}$ alkoxy, amino, $C_{1-3}$ alkylamino, or di($C_{1-3}$alkyl)amino; (b) phenyl or phen $C_{1-3}$ alkyl having from two to five $R_5$ substituents where each $R_5$, independently of the other(s), is halogen, cyano, trifluoromethyl, $C_{2-3}$ alkanoyl, nitro, $C_{1-3}$ alkyl, $C_{1-3}$ alkoxy, carboxy, alkoxycarbonyl, trifluoromethoxy, acetamido, $C_{1-3}$ alkylthio, $C_{1-3}$ alkylsulfinyl, $C_{1-3}$ alkylsulfonyl, trichlorovinyl, trifluoromethylthio, trifluoromethylsulfinyl, trifluoromethylsulfonyl or

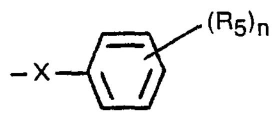

where

X and n are as defined above and $R_5$ is as defined above except that it is not

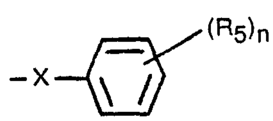

provided that if a substituent is halogen, $C_{1-3}$ alkyl or $C_{1-3}$ alkoxy, that substituent is in a position other than those *ortho* to the positions of attachment of the phenyl to the imidazole or the alkyl that is in turn attached to the imidazole; or (c) phenacyl, pyridyl, pyridylmethyl, naphthyl, naphthylmethyl, quinolyl, or quinolylmethyl;

$R_2$ is amino, $C_{1-3}$ alkylamino, di($C_{1-3}$ alkyl)amino, acetamido, acetimido, ureido, formamido, formimido or guanidino;

$R_3$ is carbamoyl, cyano, carbazoyl, amidino or N-hydroxycarbamoyl; and

$R_4$ is hydrogen, $C_{1-3}$ alkyl, hydroxy, amino, $C_{1-3}$ alkylamino, di($C_{1-3}$ alkyl)amino, phenyl, cyano, $C_{1-3}$ alkoxy, $C_{2-3}$ alkanolyoxy, $C_{1-3}$ alkylthio, $C_{1-3}$ alkylsulfinyl, or $C_{1-3}$ alkylsulfonyl. Preferably, when a given $R_5$ is defined so as to include a second $R_5$ group, then that second $R_5$ group cannot itself be defined so as to include a third $R_5$ group.

Administration of a small amount of at least one of the compounds of the invention, preferably in a composition with an inert carrier, conveniently a poultry feed, can be effective in preventing or greatly

reducing the incidence of coccidiosis. Such compositions are another aspect of the present invention. The compounds are effective against both the caecal form (caused principally by *E. tenella*) and the intestinal forms (principally caused by *E. acervulina, E. brunetti, E. maxima* and *E. necatrix*). The coccidiostats of this invention are particularly effective against the species that cause caecal damage in addition to preventing the pathology caused by the coccidia. These compounds also exert an inhibitory effect on the oocysts by greatly reducing the number and or the sporulation of those produced. They are administered in compositions that also include an inert carrier.

The compounds of the invention are also active against *Eimeria spp,* in other animals.

The novel imidazole derivatives of this invention are prepared by reacting an appropriately substituted halide and a 1-substituted imidazole compound in the presence of a base in a suitable reaction medium.

The preferred compounds of the invention are those in which, in the foregoing structural formula, $R_1$ is monosubstituted phenyl or monosubstituted benzyl where the substituent is a trifluoromethyl, phenoxy, benzoyl, phenylthio, phenylsulfinyl or phenylsulfonyl radical or a halo-substituted, methyl-substituted or trifluoromethyl-substituted phenoxy, phenylthio, phenylsulfinyl, phenylsulfonyl, benzoyl or phenylhydroxymethyl radical; a di- or trisubstituted phenyl or benzyl radical where the substituents are halogen, cyano, methyl, trifluoromethyl, phenoxy, benzoyl, phenylthio, phenylsulfinyl, phenylsulfonyl, or a halo-substituted, methyl-substituted or trifluoromethyl-substituted phenoxy, phenylthio, phenylsulfinyl, phenylsulfonyl, benzoyl or phenylhydroxymethyl radical; provided that if the monosubstituent or one of the substituents is halogen, it is in a position other than those *ortho* to the position of attachment of the phenyl to the imidazole or to the methyl that is in turn attached to the imidazole;

$R_2$ is amino, lower alkylamino or di(lower alkyl)amino;

$R_3$ is carbamoyl and

$R_4$ is hydrogen.

The especially preferred compounds of the invention are those in which $R_1$ is a phenyl or benzyl radical having 2 or 3 halo, cyano, methyl, trifluoromethyl, halophenoxy, tolyoxy, trifluoromethylphenoxy, halophenylthio, tolylthio, trifluoromethylphenylthio, halophenylsulfinyl, tolysulfinyl, trifluoromethylphenylsulfinyl, halophenylsulfonyl, tolylsulfonyl, trifluoromethylphenylsulfonyl, halobenzoyl, methylbenzoyl, trifluoromethylbenzoyl, halophenyl-hydroxymethyl, methylphenylhydroxymethyl and/or trifluoromethylphenyl-hydroxymethyl substituents in the *meta* and/or *para* positions;

$R_2$ is amino;

$R_3$ is carbamoyl; and

$R_4$ is hydrogen.

In the present specification and claims the term "lower alkyl" means "$C_{1-3}$ alkyl", viz. methyl, ethyl, propyl or isopropyl; the term "lower alkoxy" means "$C_{1-3}$ alkoxy", viz. methoxy, ethoxy, propoxy, or isopropoxy; and the term "lower alkanoyl" means $C_{2-3}$ alkanoyl, viz. acetyl and propionyl.

The compounds of the invention may be prepared by any one of several processes. The most general process is outlined in the following reaction scheme.

Reaction Scheme I

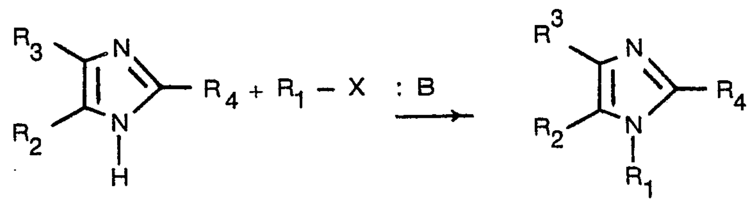

where X is a halogen preferably chlorine or bromine. In the foregoing reaction a 1-unsubstituted but otherwise appropriately substituted imidazole is reacted with a halogen substituted $R_1$ group in the presence of a base to prepare the desired 1-substituted imidazole. The reaction is carried out in a solvent which may be any polar aprotic organic solvent such as acetone, dimethylformamide, acetonitrile, dioxane, and the like in the presence of a base. The base may be any non-nucleophilic organic or inorganic base since its purpose is merely to neutralize the acid produced during the course of the reaction. Suitable inorganic bases are alkali metal bases, such as sodium and potassium carbonates, phosphates, bicarbonates and hydroxides. Suitable organic bases are tertiary amines such as trialkyl substituted amines and cyclic aromatic amines such as collidine. The reaction rate varies greatly with the nature of the proposed substituent at the $R_1$ position, the base being used in the reaction and the solvent. Very reactive substituent and base combinations may be complete in as little as ten minutes and at the other extreme the reaction may take as long as two weeks. Most reactions are however complete in from 1 to 100 hours. The reaction is carried out at a temperature of from room temperature to 100°C or to the reflux temperature of the solvent system being used. The products of the reaction are isolated using techniques known to those skilled in the art.

An alternative procedure for preparing the imidazole compounds where $R_2$ is amino and $R_3$ is carbamoyl is outlined in the following reaction scheme:

Reaction Scheme 2

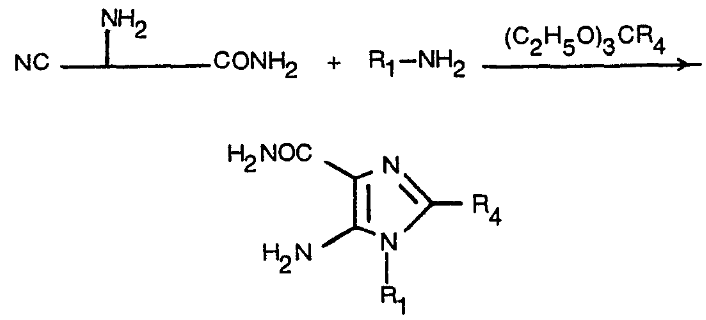


wherein $R_4$ is hydrogen, lower alkyl, or phenyl. The above reaction is carried out in a non-polar aprotic solvent system as described in the preceding reaction scheme. The reaction is carried out by first combining the aminocyanoacetamide and triethylorthoformate in the solvent and stirring at from room temperature to 100°C or to the reflux temperature of the solvent system being employed for from 10 minutes to 3 hours. Generally this phase of the reaction is complete in from ½ to 1 hour. However, following this reaction period the $R_1$ substituted amine is added to the reaction mixture and the reaction stirred for up to 2 hours at from room temperature to 100°C or the reflux temperature of the reaction system. The system is often very fast being evidenced by the immediate production of a precipitate and the product may be isolated immediately. However, generally to ensure that the reaction is complete, stirring and heating are continued for a short time. The products of the reaction are isolated using techniques known to those skilled in the art.

The novel compounds of this invention are orally administered to poultry for the control and prevention of coccidiosis. Any number of conventional methods are suitable for administering the coccidiostats of this invention to poultry, as for example, they may be given in the poultry feed. The actual quantity of the coccidiostats administered to the poultry in accordance with this invention will vary over a wide range and be adjusted to individual needs, depending upon species of the coccidia involved and severity of the infection. The limiting criteria are that the minimum amount is sufficient to control coccidiosis and the maximum amount is such that the coccidiostat does not result in any undesirable effects.

A feed typically contains from about 0.0001 to about 0.2 percent, preferably from about 0.001 to about 0.1 percent, by weight of one of the coccidiostats of this invention. The optimum levels will naturally vary with the specific compound utilized and species of *Eimeria* involved, and can be readily determined by one skilled in the art. Levels of the 5-amino and substituted amino imidazoles of this invention, in poultry feed of from about 0.001 percent to about 0.1 percent by weight of the diet are especially useful in controlling the pathology associated with *E. tenella,* as well as the intestinal dwelling species.

Depending on the compound employed, levels of 0.001 percent to 0.006 percent possess the novel effects of reducing the number of oocysts passed in the droppings of infected chickens and/or inhibiting the subsequent division and maturation to infectivity, scientifically designated as the process of sproulation. Thus, the combination of prevention of pathology, coupled with the inhibiting effect on the reproductive product of these organisms, the oocysts, present a unique two-fold method for the control of coccidiosis in poultry.

The quanitity or concentration of a novel coccidiostat of this invention in any admixture in which it is administered to the poultry will, of course, vary in accordance with the type of admixture utilized.

Of the various methods of administering the coccidiostats of this invention to poultry, they are most conveniently administered as a component of a feed composition. The novel coccidiostats may be readily dispersed by mechanically mixing the same in finely ground form with the poultry feedstuff, or with an intermediate formulation (premix) that is subsequently blended with other components to prepare the final poultry feedstuff that is fed to the poultry. Typical components of poultry feedstuffs include molasses, fermentation residues, corn meal, ground and rolled oats, wheat shorts and middlings, alfalfa, clover and meat scraps, together with mineral supplements such as bone meal and calcium carbonate and vitamins.

The following non-limiting examples will serve to illustrate the instant invention.

## Example 1

Preparation of 1-substituted-5-aminoimidazole-4-carboxamides (Method A)

A mixture of 5-aminoimidazole-4-carboxamide hydrochloride, potassium carbonate, alkyl halide, and acetone was refluxed for from 3 to 168 hours, the solvent was concentrated to about ⅙ of the original volume and the mixture was filtered. The solid was washed with acetone, slurried in water, and filtered. The remaining solid was slurried in water, treated with glacial acetic acid to remove residual potassium carbonate, and filtered. The filter cake was washed with water, acetone, and ether to provide the desired 1-substituted-5-aminoimidazole-4-carboxamide. (Table 1).

TABLE 1

| alkyl halide ($R_1$—X) (X = halogen) | Wt (g) | 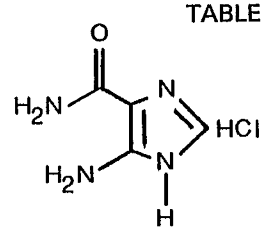 Wt (g) | $K_2CO_3$ Wt (g) | acetone Vol (ml) | reflux time (hr) | yield (g) 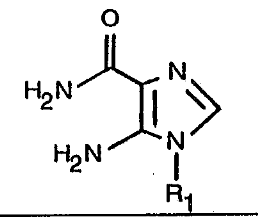 |
|---|---|---|---|---|---|---|
| 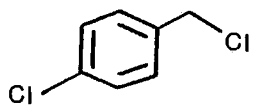 7.25 | 4.9 | 16.6 | 300 | 48 | 4.0 | 271—273[1] |
| 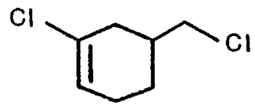 2.9 | 1.95 | 6.6 | 125 | 46 | 1.98 | 251—252[2] |
| 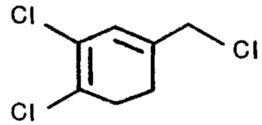 8.8 | 4.9 | 16.6 | 300 | 65 | 5.15 | 241—244 |
| 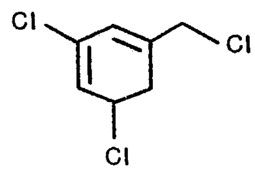 3.5 | 1.95 | 6.6 | 125 | 64 | 1.30 | 247—248[2] |
| 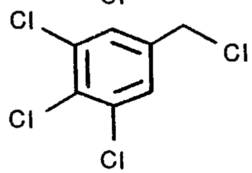 6.61 | 3.09 | 10.5 | 250 | 26 | 2.5 | 286—287.5[2] |
| 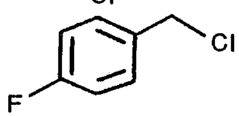 6.5 | 4.9 | 16.6 | 300 | 20 | 5.8 | 237—241 |
| 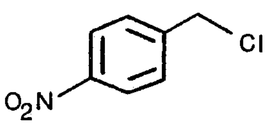 7.15 | 4.9 | 16.6 | 300 | 3 | 1.2 | 219—221 |

TABLE 1

| alkyl halide ($R_1$—X) (X = halogen) | Wt (g) | 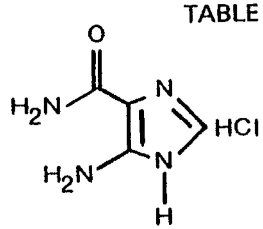 Wt (g) | $K_2CO_3$ Wt (g) | acetone Vol (ml) | reflux time (hr) | yield (g) 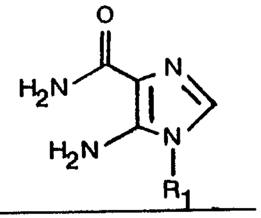 |
|---|---|---|---|---|---|---|
| 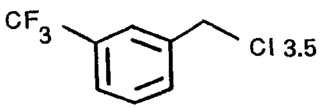 3.5 | 1.95 | 6.6 | 125 | 48 | 1.54 | 192.5—194[2] |
| 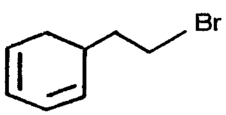 8.3 | 4.9 | 16.6 | 300 | 90 | 1.95 | 206—209 |
| 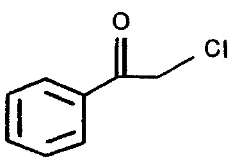 6.95 | 4.9 | 16.6 | 300 | 21 | 2.3 | 197—215 |

[1] In this case crude solid product was recrystallized from 65 ml acetic acid-water (10:3 v/v).
[2] Melting point after recrystallization from aqueous ethanol.

Other compounds which can be prepared by Method A:

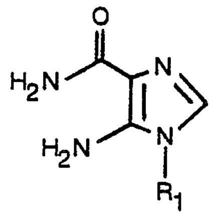

| $R_1$ | |
|---|---|
| 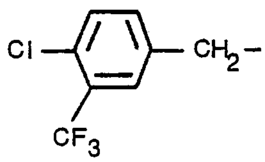 | 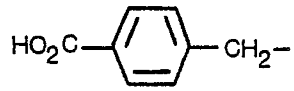 |
| 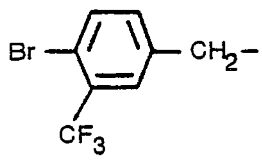 | 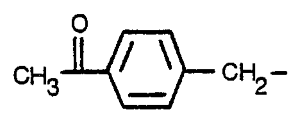 |
| 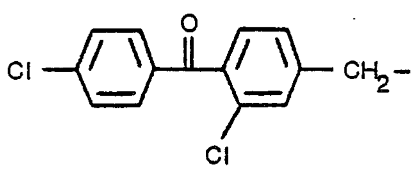 | 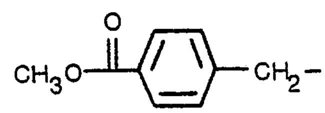 |
| | 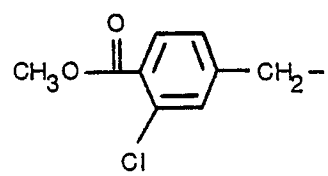 |

Example 2

Preparation of 1-substituted-5-aminoimidazole-4-carboxamides (Method B)

A mixture of aminocyanoacetamide and triethyl orthoformate in acetonitrile was refluxed for 30—55 minutes. The mixture may be filtered if a small amount of precipitate forms. A primary amine, $R_1NH_2$, was added and the mixture was refluxed for 15—30 minutes. The mixture was cooled and product collected by filtration (Table 2) or isolated by chromatography.

TABLE 2

| 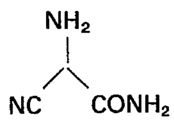 (g) | (EtO)$_3$CH (g) | $CH_3CN$ (ml) | reflux time (min.) | amine $R_1$—$NH_2$ | weight amine (g) | additional reflux time (min.) | yield (g) 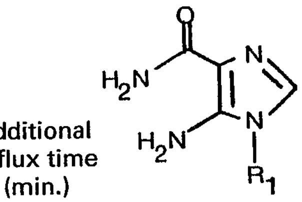 | melting point (°C) |
|---|---|---|---|---|---|---|---|---|
| 2.00 | 3.29 | 30 | 45 | 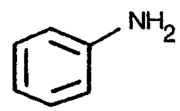 | 1.88 | 15 | 1.5 | 190—194 |
| 2.00 | 3.29 | 30 | 30 | 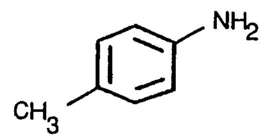 | 2.16 | 30 | 3.1 | 242.5—264 (dec) |
| 2.00 | 3.30 | 30 | 30 | 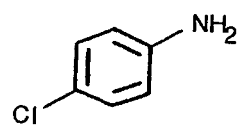 | 2.58 | 30 | 1.7 | 262—263 |
| 1.48 | 2.48 | 22 | 45 | 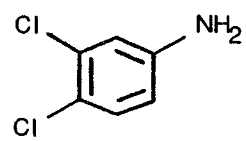 | 2.40 | 45 | 1.97 | |
| 2.56 | 4.21 | 25 | 45 | 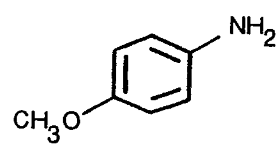 | 3.18 | 20 | 3.55 | 236—238 |
| 2.00 | 3.29 | 30 | 45 | 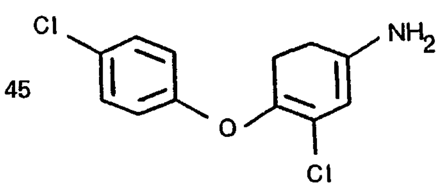 | 5.13 | 15 | 4.4 | 188—191[1] |

TABLE 2 (continued)

| 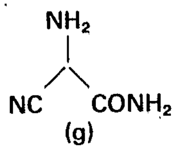 (g) | $(EtO)_3CH$ (g) | $CH_3CN$ (ml) | reflux time (min.) | amine $R_1$—$NH_2$ | weight amine (g) | additional reflux time (min.) | yield (g) 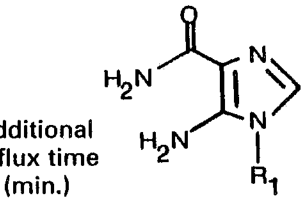 | melting point (°C) |
|---|---|---|---|---|---|---|---|---|
| 0.297 | 0.489 | 4.5 | 45 | 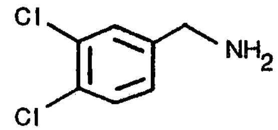 | 0.528 | 15 | 0.609 | 238—240 |
| 0.531 | 0.877 | 8.0 | 45 | 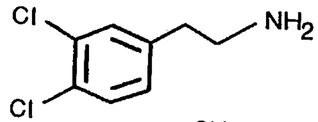 | 1.02 | 15 | 0.885 | 235—237 |
| 1.48 | 2.48 | 22 | 45 | 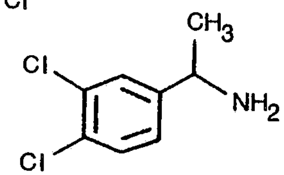 | 2.85 | 15 | 2.66 | 247—249 |
| 2.00 | 3.29 | 30 | 50 | 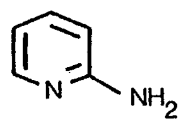 | 1.92 | 15 | 1.05 | 198.5—200[1] |
| 0.273 | 0.454 | 4.1 | 45 | 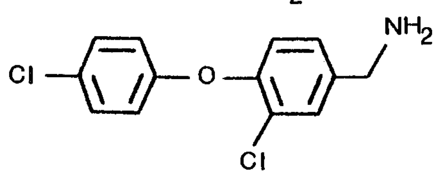 | 0.751 | 15 | 0.340 | 199—200 |
| 1.24 | 2.05 | 18 | 45 | 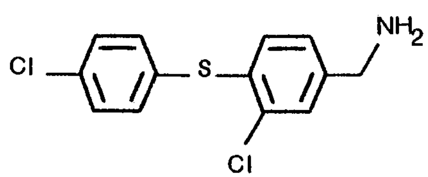 | 3.6 | 15 | 3.16 | 201—202 |

TABLE 2 (continued)

| 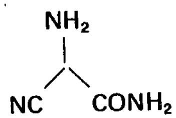 (g) | (EtO)$_3$CH (g) | $CH_3CN$ (ml) | reflux time (min.) | amine $R_1$—$NH_2$ | weight amine (g) | additional reflux time (min.) | yield (g) 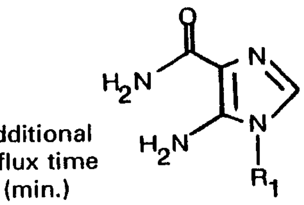 | melting point (°C) |
|---|---|---|---|---|---|---|---|---|
| 0.500 | 0.820 | 8.0 | 45 | 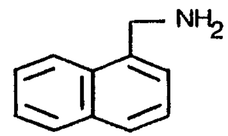 | 0.785 | 15 | 0.831 | 258—260 |
| 0.870 | 1.43 | 14 | 45 | 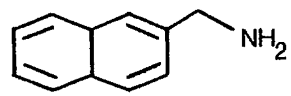 | 1.38 | 15 | 1.62 | 264—270 |
| 0.700 | 1.25[2] | 12 | 45 | 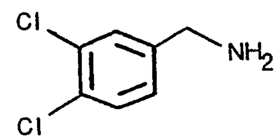 | 1.24 | 15 | 1.22[3] | 275 |
| 0.556 | 0.89 | 8.0 | 45 | 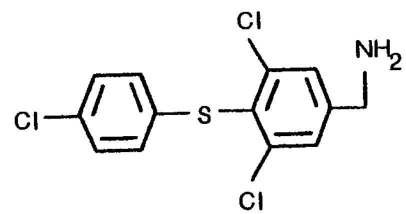 | 1.79 | 15 | 1.24 | 221—222 |
| 0.314 | 0.49 | 4.0 | 45 | 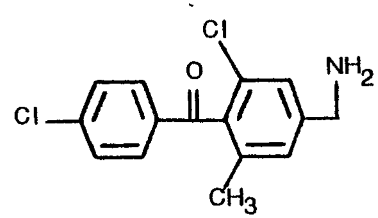 | 0.845 | 15 | 0.741 | 229—230 |

TABLE 2 (continued)

| 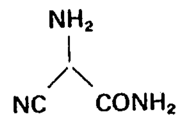 (g) | (EtO)₃CH (g) | CH₃CN (ml) | reflux time (min.) | amine $R_1$—$NH_2$ | weight amine (g) | additional reflux time (min.) | yield (g) 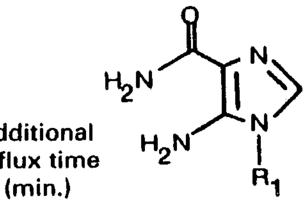 | melting point (°C) |
|---|---|---|---|---|---|---|---|---|
| 0.332 | 0.52 | 4.2 | 45 | 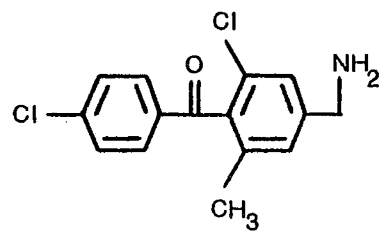 | 0.830 | 30 | 0.592 | 226—227 |
| 0.117 | 0.183 | 1.5 | 45 | 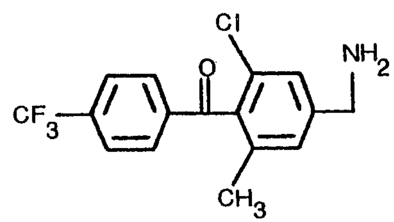 | 0.350 | 45 | 0.121 | 221—223 |

[1] Melting point after recrystallization from 98:2 (v/v) ethanol-benzene.
[2] Weight of triethylorthoacetate.
[3] Yield of 1-(3,4-dichlorobenzyl)-2-methyl-5-aminoimidazole-4-carboxamide, isolated in two crops, triturated with 20 ml of hot acetonitrile and dried.

Other compounds which can be prepared by Method B:

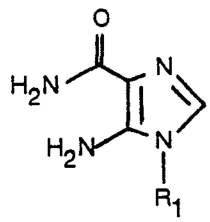

| $R_1$ | $R_1$ | $R_1$ |
| --- | --- | --- |
| 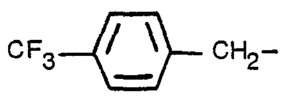 | 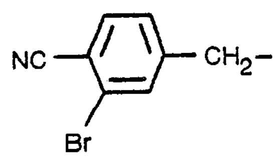 | 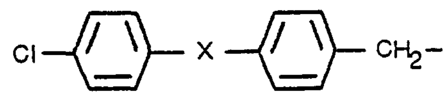<br>X = SO, $SO_2$ |
| 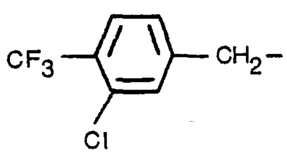 | 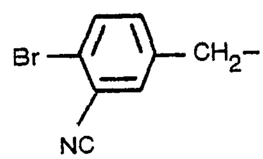 | |
| 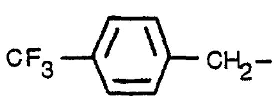 | 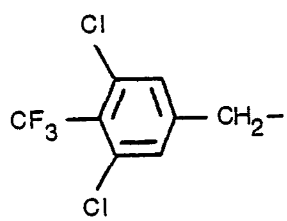 | |
| 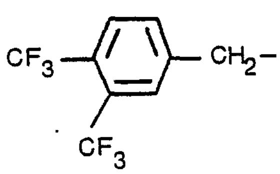 | 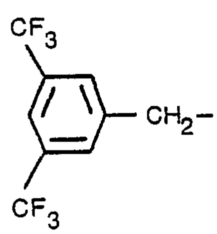 | |

| $R_1$ | $R_1$ | $R_1$ |
|---|---|---|
| 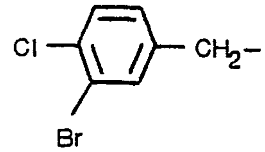 | 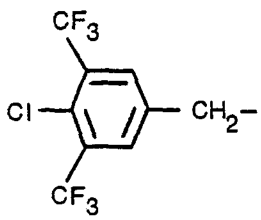 | |
| 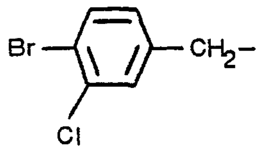 | 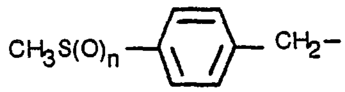 n = 0, 1, 2 | |
| 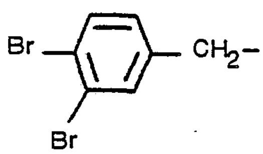 | 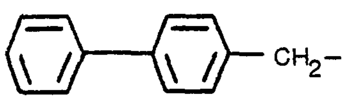 | |
| 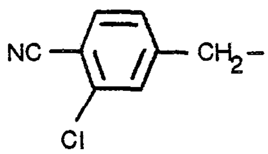 | | |
| 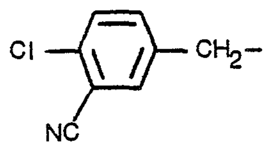 | | |
| 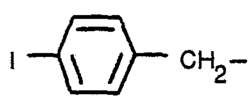 | 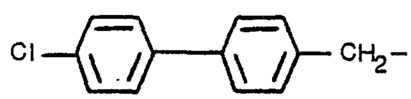 | |

Example 3

Preparation of 1-(m-cyanobenzyl)-5-aminoimidazole-4-carboxamide

A mixture of 5-aminoimidazole-4-carboxamide (5.00 g), potassium carbonate (12.0 g), and α-bromo-*m*-tolunitrile (9.80 g) was refluxed in acetone (300 ml) for 24 hours under a nitrogen atmosphere. The mixture was cooled to room temperature and filtered. The solid residue was washed with acetone and the combined filtrates were evaporated to dryness. The residual solid was dissolved in acetone (50 ml), concentrated to a volume of 20 ml *in vacuo,* and diluted with diethyl ether (100 ml) to provide a gum. The solvent was decanted from the residue and deposited crystals of crude product on standing. The gum was triturated twice with acetone, and the acetone layers were combined with the above crystals, and evaporated to provide 5.9 g of a dark gum. The gum was dissolved in methanol (100 ml), filtered, added to 100 ml. E. Merck 7734 silica gel, and evaporated to dryness *in vacuo.* The product on silica gel was placed on top of a column of 1200 ml E. Merck 7734 silica gel and eluted with 9:1 v/v methylene chloride/methanol. After a forerun of 1.0 l, 400 ml fractions were collected and fractions 8—11 and 12—15 were combined separately and evaporated to dryness. The solid product from fractions 8—11 was triturated with a small volume of acetone and filtered. The filtrate was combined separately with the product from fractions

12—15 and evaporated to dryness. The product was recrystallized from methanol to provide 320 mg of 1-(*m*-cyanobenzyl)-5-aminoimidazole-4-carboxamide, m.p. 246—247°C.

Example 4

Preparation of 1-(4-chloro-3-trifluoromethylbenzyl)-5-aminoimidazole-4-carboxamide

A mixture of 5-aminoimidazole-4-carboxamide hydochloride (5.0 g), $K_2CO_3$ (16.5 g), and a 9:1 w/w mixture of α,4-chloro-3-trifluoromethyltoluene and α,2-dichloro-3-trifluoromethyltoluene was refluxed in acetone (300 ml) for 4 days. Solvent was concentrated *in vacuo,* the residue was diluted with water, and the solution was extracted with ethyl acetate. The combined ethyl acetate extracts were washed with brine, 0.5 *N* acetic acid, and brine, dried, treated with activated charcoal, and filtered. The filtrate was concentrated to provide a first crop of 3.40 g. The filtrate was diluted with ether to provide a second crop of 2.07 g, and the remaining filtrate was diluted with hexane to provide a third crop of 0.25 g. The second and third crops were combined, dissolved in aqueous ethanol, diluted with water, and concentrated to provide 0.97 g of solid. Further concentration of the filtrate provided an additional 0.43 g. The samples weighing 3.40 g, 0.97 g, and 0.43 g were combined, treated with hot 7.5% methanol in ethyl acetate, and diluted with 50 ml ethyl acetate. The resulting solution was chromatographed on a column of 500 ml of silica gel, eluted with 7.5% methanol in ethyl acetate followed by 10% methanol in ethyl acetate. A total of 150 fractions of 20 ml each were collected at a flow rate of about 10 ml/min. Fractions 60—118 were combined and evaporated to provide 3.53 g solid. The product was dissolved in 100 ml of boiling ethanol, treated with activated charcoal, and filtered. The filtrate was concentrated to provide a first crop of crystals and further concentration of the filtrate provided a second crop. The two crops were combined and recrystallized from 50 ml of ethanol to provide 1.99 g 1-(4-chloro-3-trifluoromethylbenzyl)-5-aminoimidazole-4-carboxamide, m.p. 230.5—232.5°C.

**Claims for the Contracting States: BE CH DE FR GB IT LI LU NL SE**

1. A compound having the formula:

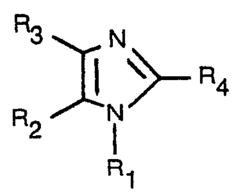

in which $R_1$ is (a) mono-substituted phenyl or mono-substituted phen $C_{1-3}$ alkyl where the substituent is trifluoromethyl, $C_{2-3}$ alkanoyl, nitro, carboxy, alkoxycarbonyl, acetamido, $C_{1-3}$ alkylthio, $C_{1-3}$ alkylsulfinyl, $C_{1-3}$ alkylsulfonyl or

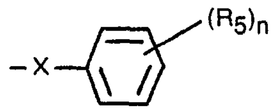

where n is from 0 to 5, $R_5$ is as defined below and X is O, S, SO, $SO_2$, $CH_2$, CO, CHOH, CHCN or $C{=}NR_6$ where $R_6$ is hydrogen, $C_{1-3}$ alkyl, hydroxy, $C_{1-3}$ alkoxy, amino, $C_{1-3}$ alkylamino, or di($C_{1-3}$ alkyl)amino; (b) phenyl or phen $C_{1-3}$ alkyl having from two to five $R_5$ substituents where each $R_5$, independently of the other(s), is halogen, cyano, trifluoromethyl, $C_{2-3}$ alkanoyl, nitro, $C_{1-3}$ alkyl, $C_{1-3}$ alkoxy, carboxy, alkoxycarbonyl, trifluoromethoxy, acetamido, $C_{1-3}$ alkylthio, $C_{1-3}$ alkylsulfinyl, $C_{1-3}$ alkylsulfonyl, trichlorovinyl, trifluoromethylthio, trifluoromethylsulfinyl, trifluoromethylsulfonyl or

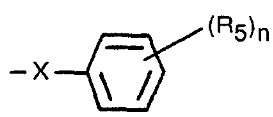

where

X and n are as defined above and $R_5$ is as defined above except that it is not

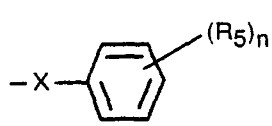

provided that if a substituent is halogen, $C_{1-3}$ alkyl or $C_{1-3}$ alkoxy, that substituent is in a position other than those *ortho* to the positions of attachment of the phenyl to the imidazole or the alkyl that is in turn attached to the imidazole; or (c) phenacyl, pyridyl, pyridylmethyl, naphthyl, naphthylmethyl, quinolyl, or quinolylmethyl;

$R_2$ is amino, $C_{1-3}$ alkylamino, di($C_{1-3}$ alkyl)amino, acetamido, acetimido, ureido, formamido, formimido or guanidino;

$R_3$ is carbamoyl, cyano, carbazoyl, amidino or N-hydroxycarbamoyl; and

$R_4$ is hydrogen, $C_{1-3}$ alkyl, hydroxy, amino, $C_{1-3}$ alkylamino, di($C_{1-3}$ alkyl)amino, phenyl, cyano, $C_{1-3}$ alkoxy, $C_{2-3}$ alkanoyloxy, $C_{1-3}$ alkylthio, $C_{1-3}$ alkylsulfinyl, or $C_{1-3}$ alkylsulfonyl.

2. A compound as claimed in Claim 1, in which $R_1$ is monosubstituted phenyl or monosubstituted benzyl where the substituent is a trifluoromethyl, phenoxy, benzoyl, phenylthio, phenylsulfinyl or phenylsulfonyl radical or a halo-substituted, methyl-substituted or trifluoromethyl-substituted phenoxy, phenylthio, phenylsulfinyl, phenylsulfonyl, benzoyl or phenylhydroxymethyl radical; a di- or trisubstituted phenyl or benzyl radical where the substituents are halogen, cyano, methyl, trifluoromethyl, phenoxy, benzoyl, phenylthio, phenylsulfinyl, phenylsulfonyl, or a halo-substituted, methyl-substituted or trifluoromethyl-substituted phenoxy, phenylthio, phenylsulfinyl, phenylsulfonyl, benzoyl or phenylhydroxymethyl radical; provided that if a substituent is halogen, it is in a position other than those *ortho* to the position of attachment of the phenyl to the imidazole or to the methyl that is in turn attached to the imidazole; $R_2$ is amino, $C_{1-3}$ alkylamino or di($C_{1-3}$ alkyl)amino; $R_3$ is carbamoyl and $R_4$ is hydrogen.

3. A compound as claimed in Claim 2, in which $R_1$ is a phenyl or benzyl radical having 2 or 3 halo, cyano, methyl, trifluoromethyl, halophenoxy, tolyoxy, trifluoromethylphenoxy, halophenylthio, tolylthio, trifluoromethylphenylthio, halophenylsulfinyl, tolysulfinyl, trifluoromethylphenylsulfinyl, halophenylsulfonyl, tolylsulfonyl, trifluoromethylphenylsulfonyl, halobenzoyl, methylbenzoyl, trifluoromethylbenzoyl, halophenyl-hydroxymethyl, methylphenylhydroxymethyl and/or trifluoromethylphenyl-hydroxymethyl substituents in the *meta* and/or *para* positions; $R_2$ is amino; $R_3$ is carbamoyl; and $R_4$ is hydrogen.

4. 5-Amino-1-(3,4,5-trichlorobenzyl)imidazole-4-carboxamide.

5. 5-Amino-[4-(4-chlorophenylthio)-3-chlorobenzyl]imidazole-4-carboxamide.

6. 5-Amino-1-[4-(4-chlorophenoxy)-3-chlorobenzyl]imidazole-4-carboxamide.

7. A process for the preparation of a compound as claimed in Claim 1 comprising reacting a compound having the formula

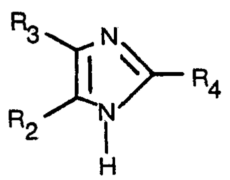

with an $R_1$-substituted halide in the presence of a base to produce the desired compound, $R_1$, $R_2$, $R_3$ and $R_4$ being as defined in Claim 1.

8. A process for the preparation of a compound as claimed in Claim 1 and having the formula

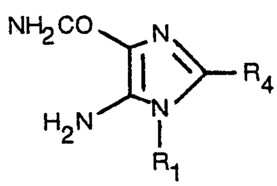

comprising treating aminocyanoacetamide with an $R_1$-substituted amine in the presence of a compound of formula $(C_2H_5O)_3CR_4$ to produce the desired compound, $R_4$ being hydrogen, $C_{1-3}$ alkyl, or phenyl and $R_1$ being as defined in Claim 1.

9. A compound as claimed in any one of Claims 1 to 6 for use in administration to an animal for the purpose of preventing or treating coccidiosis.

10. A composition useful for the prevention and treatment of coccidiosis which comprises an inert carrier and a compound as claimed in any one of Claims 1 to 6.

**Claims for the Contracting State: AT**

1. A process for preparing a compound of formula:

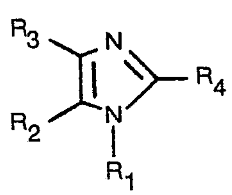

comprising reacting a compound having the formula

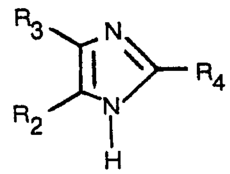

with an $R_1$-substituted halide in the presence of a base to produce the desired compound, where in the formula, $R_1$ is (a) mono-substituted phenyl or mono-substituted phen $C_{1-3}$ alkyl where the substituent is trifluoromethyl, $C_{2-3}$ alkanoyl, nitro, carboxy, alkoxycarbonyl, acetamido, $C_{1-3}$ alkylthio, $C_{1-3}$ alkylsulfinyl, $C_{1-3}$ alkylsulfonyl or

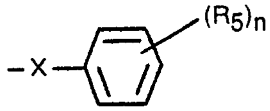

where n is from 0 to 5, $R_5$ is as defined below and X is O, S, SO, $SO_2$, $CH_2$, CO, CHOH, CHCN or $C{=}NR_6$ where $R_6$ is hydrogen, $C_{1-3}$ alkyl, hydroxy, $C_{1-3}$ alkoxy, amino, $C_{1-3}$ alkylamino, or di($C_{1-3}$ alkyl)amino; (b) phenyl or phen $C_{1-3}$ alkyl having from two to five $R_5$ substituents where each $R_5$, independently of the other(s), is halogen, cyano, trifluoromethyl, $C_{2-3}$ alkanoyl, nitro, $C_{1-3}$ alkyl, $C_{1-3}$ alkoxy, carboxy, alkoxycarbonyl, trifluoromethoxy, acetamido, $C_{1-3}$ alkylthio, $C_{1-3}$ alkylsulfinyl, $C_{1-3}$ alkylsulfonyl, trichlorovinyl, trifluoromethylthio, trifluoromethylsulfinyl, trifluoromethylsulfonyl or

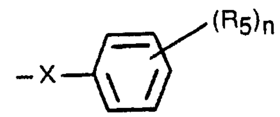

where
X and n are as defined above and $R_5$ is as defined above except that it is not

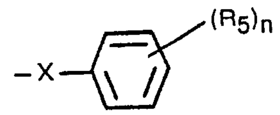

provided that if a substituent is halogen, $C_{1-3}$ alkyl or $C_{1-3}$ alkoxy, that substituent is in a position other than those *ortho* to the positions of attachment of the phenyl to the imidazole or the alkyl that is in turn attached to the imidazole; or (c) phenacyl, pyridyl, pyridylmethyl, naphthyl, naphthylmethyl, quinolyl, or quinolylmethyl;

$R_2$ is amino, $C_{1-3}$ alkylamino, di($C_{1-3}$ alkyl)amino, acetamido, acetimido, ureido, formamido, formimido or guanidino;

$R_3$ is carbamoyl, cyano, carbazoyl, amidino or N-hydroxycarbamoyl; and

$R_4$ is hydrogen, $C_{1-3}$ alkyl, hydroxy, amino, $C_{1-3}$ alkylamino, di($C_{1-3}$ alkyl)amino, phenyl, cyano, $C_{1-3}$ alkoxy, $C_{2-3}$ alkanoyloxy, $C_{1-3}$ alkylthio, $C_{1-3}$ alkylsulfinyl, or $C_{1-3}$ alkylsulfonyl.

2. A process for preparing a compound having the formula

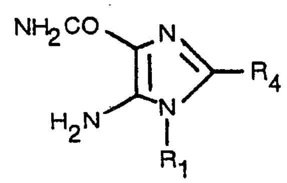

comprising treating aminocyanoacetamide with an $R_1$-substituted amine in the presence of a compound of formula $(C_2H_5O)_3CR_4$ to produce the desired compound, $R_4$ being hydrogen, $C_{1-3}$ alkyl, or phenyl and $R_1$ being as defined in Claim 1.

3. A process as claimed in Claim 1 as applied to the preparation of a compound in which $R_1$ is monosubstituted phenyl or monosubstituted benzyl where the substituent is a trifluoromethyl, phenoxy, benzoyl, phenylthio, phenylsulfinyl or phenylsulfonyl radical, a halo-substituted, methyl-substituted or trifluoromethyl-substituted phenoxy, phenylthio, phenylsulfinyl, phenylsulfonyl, benzoyl or phenylhydroxymethyl radical; a di- or trisubstituted phenyl or benzyl radical where the substituents are halogen, cyano, methyl, trifluoromethyl, phenoxy, benzoyl, phenylthio, phenylsulfinyl, phenylsulfonyl, or a halo-substituted, methyl-substituted or trifluoromethyl-substituted phenoxy, phenylthio, phenylsulfinyl, phenylsulfonyl, benzoyl or phenylhydroxymethyl radical; provided that if a substituent is halogen, it is in a position other than those *ortho* to the position of attachment of the phenyl to the imidazole or to the methyl that is in turn attached to the imidazole; $R_2$ is amino, $C_{1-3}$ alkylamino or di($C_{1-3}$ alkyl)amino; $R_3$ is carbamoyl and $R_4$ is hydrogen.

4. A process as claimed in Claim 2 as applied to the preparation of a compound in which $R_1$ is as defined in Claim 3 and $R_4$ is hydrogen.

5. A process as claimed in Claim 1 or 2 as applied to the preparation of a compound in which $R_1$ is a phenyl or benzyl radical having 2 or 3 halo, cyano, methyl, trifluoromethyl, halophenoxy, tolyoxy, trifluoromethylphenoxy, halophenylthio, tolylthio, trifluoromethylphenylthio, halophenylsulfinyl, tolysulfinyl, trifluoromethylphenylsulfinyl, halophenylsulfonyl, tolylsulfonyl, trifluoromethylphenylsulfonyl, halobenzoyl, methylbenzoyl, trifluoromethylbenzoyl, halophenyl-hydroxymethyl, methylphenylhydroxymethyl and/or trifluoromethylphenyl-hydroxymethyl substituents in the *meta* and/or *para* positions; and $R_4$ is hydrogen.

6. A process as claimed in Claim 1 or 2 as applied to the preparation of 5-amino-1-(3,4,5-trichlorobenzyl)imidazole-4-carboxamide.

7. A process as claimed in Claim 1 or 2 as applied to the preparation of 5-amino-1-[4-(4-chlorophenylthio)-3-chlorobenzyl]imidazole-4-carboxamide.

8. A process as claimed in Claim 1 or 2 as applied to the preparation of 5-amino-1-[4-(4-chlorophenoxy)-3-chlorobenzyl]imidazole-4-carboxamide.

9. A compound obtained by a process as claimed in any one of Claims 1 to 8 for use in administration to an animal for the purpose of preventing or treating coccidiosis.

10. A composition useful for the prevention and treatment of coccidiosis which comprises an inert carrier and a compound obtained by a process as claimed in any one of Claims 1 to 8.

**Patentansprüche für die Vertragsstaaten: BE CH DE FR GB IT LI LU NL SE**

1. Verbindung mit der Formel

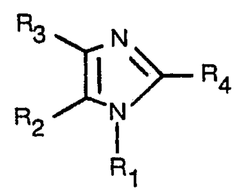

worin $R_1$ (a) mono-substituiertes Phenyl oder mono-substituiertes Phen-$C_{1-3}$-alkyl ist, worin der Substituent Trifluoromethyl, $C_{2-3}$-Alkanoyl, Nitro, Carboxy, Alkoxycarbonyl, Acetamido, $C_{1-3}$-Alkylthio, $C_{1-3}$-Alkylsulfinyl, $C_{1-3}$-Alkylsulfonyl oder

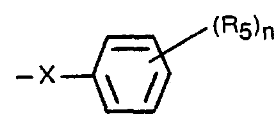

ist, worin n 0 bis 5 ist, $R_5$ wie unten definiert ist und X O, S, SO, $SO_2$, $CH_2$, CO, CHOH, CHCN oder $C{=}NR_6$ ist, worin $R_6$ Wasserstoff, $C_{1-3}$-Alkyl, Hydroxy, $C_{1-3}$-Alkoxy, Amino, $C_{1-3}$-Alkylamino oder Di($C_{1-3}$-alkyl)-amino ist; (b) Phenyl oder Phen-$C_{1-3}$-alkyl mit 2 bis 5 $R_5$-Substituenten ist, worin jedes $R_5$ unabhängig von dem/den anderen Halogen, Cyano, Trifluoromethyl, $C_{2-3}$-Alkanoyl, Nitro, $C_{1-3}$-Alkyl, $C_{1-3}$-Alkoxy, Carboxy, Alkoxycarbonyl, Trifluoromethoxy, Acetamido, $C_{1-3}$-Alkylthio, $C_{1-3}$-Alkylsulfinyl, $C_{1-3}$-Alkylsulfonyl, Trichlorvinyl, Trifluormethylthio, Trifluormethylsulfinyl, Trifluormethylsulfonyl oder

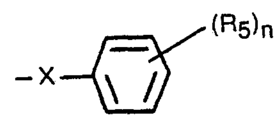

ist, worin X und n wie oben definiert sind und $R_5$ wie oben definiert ist, ausser, dass es nicht

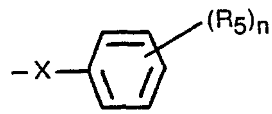

ist, vorausgesetzt, dass, falls ein Substituent Halogen, $C_{1-3}$-Alkyl oder $C_{1-3}$-Alkoxy ist, dieser Substituent in einer anderen Stellung ist als denen *ortho* zu den Stellungen der Bindung des Phenyl an das Imidazol oder das Alkyl, das statt dessen an das Imidazol gebunden ist; oder (c) Phenacyl, Pyridyl, Pyridylmethyl, Naphthyl, Naphthylmethyl, Chinolyl, oder Chinolylmethyl ist;

$R_2$ Amino, $C_{1-3}$-Alkylamino, Di($C_{1-3}$-alkyl)amino, Acetamido, Acetimido, Ureido, Formamido, Formimido oder Guanidino ist;

$R_3$ Carbamoyl, Cyano, Carbazoyl, Amidino oder N-Hydroxycarbamoyl ist; und

$R_4$ Wasserstoff, $C_{1-3}$-Alkyl, Hydroxy, Amino, $C_{1-3}$-Alkylamino, Di($C_{1-3}$-alkyl)amino, Phenyl, Cyano, $C_{1-3}$-Alkoxy, $C_{2-3}$-Alkanoyloxy, $C_{1-3}$-Alkylthio, $C_{1-3}$-Alkylsulfinyl oder $C_{1-3}$-Alkylsulfonyl ist.

2. Verbindung nach Anspruch 1, worin $R_1$ mono-substituiertes Phenyl oder mono-substituiertes Benzyl ist, worin der Substituen t ein Trifluormethyl-, Phenoxy-, Benzoyl-, Phenylthio-, Phenylsulfinyl- oder PhenylsulfonylRadikal oder ein halogen-substituiertes, methyl-substituiertes oder trifluormethyl-substituiertes Phenoxy-, Phenylthio, Phenylsulfinyl-, Phenylsulfonyl-, Benzoyl- oder Phenylhydroxymethyl-Radikal ist; ein di- oder tri-substituiertes Phenyl- oder Benzyl-Radikal ist, worin die Substituenten Halogen, Cyano, Methyl, Trifluormethyl, Phenoxy, Benzoyl, Phenylthio, Phenylsulfinyl, Phenylsulfonyl oder ein halogen-substituiertes, methyl-substituiertes oder trifluormethyl-substituiertes Phenoxy-, Phenylthio-, Phenylsulfinyl-, Phenylsulfonyl-, Benzoyl- oder Phenylhydroxymethyl-Radikal sind; vorausgesetzt, dass, falls ein Substituent Halogen ist, er in einer anderen Stellung als denen *ortho* zu der Stellung der Bindung des Phenyls an das Imidazol oder an das Methyl, das statt dessen an das Imidazol gebunden ist, vorliegt; $R_2$ Amino, $C_{1-3}$-Alkylamino oder Di($C_{1-3}$-alkyl)amino ist; $R_3$ Carbamoyl ist und $R_4$ Wasserstoff ist.

3. Verbindung nach Anspruch 2, worin $R_1$ ein Phenyl- oder Benzyl-Radikal mit 2 oder 3 Halogen-, Cyano-, Methyl-, Trifluormethyl-, Halogenphenoxy-, Tolyloxy-, Trifluormethylphenoxy-, Halogenphenylthio-, Tolylthio-, Trifluormethylphenylthio-, Halogenphenylsulfinyl-, Tolylsulfinyl-, Trifluormethylphenylsulfinyl-, Halogenphenylsulfonyl-, Tolylsulfonyl-, Trifluormethylsulfonyl-, Halogenbenzoyl-, Methylbenzoyl-, Trifluormethylbenzoyl-, Halogenphenyl-hydroxymethyl-, Methylphenyl-hydroxymethyl- und/oder Trifluormethylphenyl-hydroxymethyl-Substituenten in den *meta* und/oder *para*-Stellungen ist; $R_2$ Amino ist; $R_3$ Carbamoyl ist; und $R_4$ Wasserstoff ist.

4. 5-Amino-1-(3,4,5-trichlorbenzyl)imidazol-4-carboxamid.

5. 5-Amino-1-[4-(4-chlorphenylthio)-3-chlorbenzyl]imidazol-4-carboxamid.

6. 5-Amino-1-[4-(4-chlorphenoxy)-3-chlorbenzyl)imidazol-4-carboxamid.

7. Verfahren zur Herstellung einer Verbindung nach Anspruch 1 durch Reagieren einer Verbindung mit der Formel

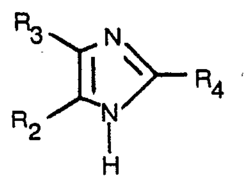

mit einem $R_1$-substituierten Halogenid in Gegenwart einer Base zur Herstellung der erwünschten Verbindung, wobei $R_1$, $R_2$, $R_3$ und $R_4$ wie in Anspruch 1 definiert sind.

8. Verbindung zur Herstellung einer Verbindung, wie in Anspruch 1 beansprucht und mit der Formel

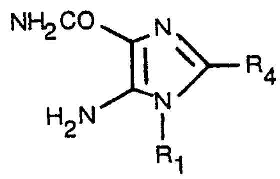

durch Behandeln von Aminocyanoacetamid mit einem $R_1$-substituierten Amin in Gegenwart einer Verbindung der Formel $(C_2H_5O)_3CR_4$ zur Herstellung der erwünschten Verbindung, wobei $R_4$ Wasserstoff, $C_{1-3}$-Alkyl oder Phenyl ist und $R_1$ wie in Anspruch 1 definiert ist.

9. Verbindung nach einem der Ansprüche 1 bis 6 zur Verwendung bei der Verabreichung an ein Tier zum Zweck der Vorbeugung oder Behandlung von Coccidiose.

10. Zusammensetzung, geeignet zur Vorbeugung und Behandlung von Coccidiose, welche einen inerten Träger und eine Verbindung, wie in einem der Ansprüche 1 bis 6 beansprucht, umfaßt.

**Patentansprüche für den Vertragsstaat: AT**

1. Ein Verfahren zur Herstellung einer Verbindung der Formel

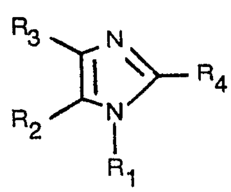

,

umfassend das Umsetzen einer Verbindung der Formel

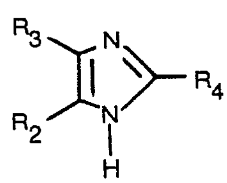

mit einem $R_1$-substituierten Halogenid in Gegenwart einer Base zur Erzeugung der gewünschten Verbindung, wobei in der Formel $R_1$ (a) mono-substituiertes Phenyl oder mono-substituiertes Phen-$C_{1-3}$-alkyl, wobei der Substituent Trifluormethyl, $C_{2-3}$-Alkanoyl, Nitro, Carboxy, Alkoxycarbonyl, Acetamido, $C_{1-3}$-Alkylthio, $C_{1-3}$-Alkylsulfinyl, $C_{1-3}$-Alkylsulfonyl oder

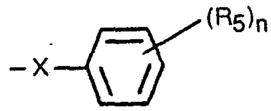,

worin n 0 bis 5 darstellt, ist, $R_5$ wie nachstehend definiert ist, und X O, S, SO, $SO_2$, $CH_2$, CO, CHOH, CHCN oder C=$NR_6$, wobei $R_6$ Wasserstoff, $C_{1-3}$-Alkyl, Hydroxy, $C_{1-3}$-Alkoxy, Amino, $C_{1-3}$-Alkylamino oder Di($C_{1-3}$-alkyl)amino ist, bedeutet; (b) Phenyl oder Phen-$C_{1-3}$-alkyl mit zwei bis fünf $R_5$-Substituenten, wobei jeder Rest $R_5$, unabhängig von dem bzw. den anderen, Halogen, Cyano, Trifluormethyl, $C_{2-3}$-Alkanoyl, Nitro, $C_{1-3}$-Alkyl, $C_{1-3}$-Alkoxy, Carboxy, Alkoxycarbonyl, Trifluormethoxy, Acetamido, $C_{1-3}$-Alkylthio, $C_{1-3}$-Alkylsulfinyl, $C_{1-3}$-Alkylsulfonyl, Trichlorvinyl, Trifluormethylthio, Trifluormethylsulfinyl, Trifluormethylsulfonyl oder

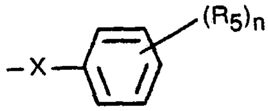

ist, wobei X und n wie oben definiert sind, und $R_5$ wie oben definiert ist, mit der Ausnahme, daß es nicht

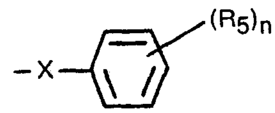

ist, mit der Maßgabe, daß, falls ein Substituent Halogen, $C_{1-3}$-Alkyl oder $C_{1-3}$-Alkoxy ist, dieser Substituent in einer Stellung vorliegt, die von jenen in ortho zu den Bindungsstellen des Phenyls an das Imidazol oder das Alkyl, das wieder an das Imidazol gebunden ist, verschieden ist; oder (c) Phenacyl, Pyridyl, Pyridylmethyl, Naphthyl, Naphthylmethyl, Chinolyl oder Chinolylmethyl; bedeutet;

$R_2$ Amino, $C_{1-3}$-Alkylamino, Di($C_{1-3}$-alkyl)amino, Acetamido, Acetimido, Ureido, Formamido, Formimido oder Guanidino ist;

$R_3$ Carbamoyl, Cyano, Carbazoyl, Amidino oder N-Hydroxycarbamoyl ist; und

$R_4$ Wasserstoff, $C_{1-3}$-Alkyl, Hydroxy, Amino, $C_{1-3}$-Alkylamino, Di($C_{1-3}$-alkyl)amino, Phenyl, Cyano, $C_{1-3}$-Alkoxy, $C_{2-3}$-Alkanoyloxy, $C_{1-3}$-Alkylthio, $C_{1-3}$-Alkylsulfinyl oder $C_{1-3}$-Alkylsulfonyl ist.

2. Ein Verfahren zur Herstellung einer Verbindung der Formel

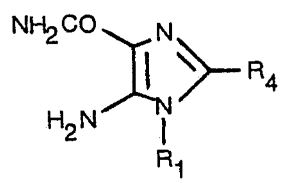,

umfassend das Behandeln von Aminocyanoacetamid mit einem $R_1$-substituierten Amin in Gegenwart einer Verbindung der Formel $(C_2H_5O)_3CR_4$ zur Erzeugung der gewünschten Verbindung, wobei $R_4$ Wasserstoff, $C_{1-3}$-Alkyl oder Phenyl ist, und $R_1$ wie in Anspruch 1 definiert ist.

3. Ein Verfahren wie in Anspruch 1 beansprucht, angewendet bei der Herstellung einer Verbindung, in welcher $R_1$ mono-substituiertes Phenyl oder mono-substituiertes Benzyl ist, wobei der Substituent ein Trifluormethyl-, Phenoxy-, Benzoyl-, Phenylthio-, Phenylsulfinyl- oder Phenylsulfonylrest, ein Halogen-substituierter, methyl-substituierter oder Trifluormethyl-substituierter Phenoxy-, Phenylthio, Phenylsulfinyl-, Phenylsulfonyl-, Benzoyl- oder Phenylhydroxymethylrest ist; oder ein di- oder tri-substituierter Phenyl- oder Benzylrest ist, wobei die Substituenten Halogen, Cyano, Methyl, Trifluormethyl, Phenoxy, Benzoyl, Phenylthio, Phenylsulfinyl, Phenylsulfonyl, oder ein Halogen-substituierter, methyl-substituierter oder Trifluormethyl-substituierter Phenoxy-, Phenylthio-, Phenylsulfinyl-, Phenylsulfonyl-, Benzoyl- oder Phenylhydroxymethylrest sind; mit der Maßgabe, daß, falls ein Substituent Halogen ist, er in einer Stellung vorliegt, die von jenen in ortho- Stellung zu der Bindungsstelle des Phenyls an das Imidazol oder an das Methyl, das wieder an das Imidazol gebunden ist, verschieden sind; $R_2$ Amino, $C_{1-3}$-Alkylamino oder Di($C_{1-3}$-alkyl)amino ist; $R_3$ Carbamoyl ist und $R_4$ Wasserstoff ist.

4. Ein Verfahren wie in Anspruch 2 beansprucht, angewendet bei der Herstellung einer Verbindung, in welcher $R_1$ wie in Anspruch 3 definiert ist, und $R_4$ Wasserstoff ist.

5. Ein Verfahren wie in Anspruch 1 oder 2 beansprucht, angewendet bei der Herstellung einer Verbindung, in welcher $R_1$ ein Phenyl- oder Benzylrest ist, der 2 oder 3 Halogen-, Cyano-, Methyl-, Trifluormethyl-, Halogenphenoxy-, Tolyloxy-, Trifluormethylphenoxy-, Halogenphenylthio-, Tolylthio-, Trifluormethylphenylthio-, Halogenphenylsulfinyl-, Tolylsulfinyl-, Trifluormethylphenylsulfinyl-, Halogenphenylsulfonyl-, Tolylsulfonyl-, Trifluormethylphenylsulfonyl-, Halogenbenzoyl-, Methylbenzoyl-, Trifluormethylbenzoyl-, Halogenphenyl-hydroxymethyl-, Methylphenyl-hydroxymethyl- und/oder Trifluormethylphenyl-hydroxymethyl-Substituenten in den meta und/oder para-Stellungen aufweist; und worin $R_4$ Wasserstoff ist.

6. Ein Verfahren wie in Anspruch 1 oder 2 beansprucht, angewendet bei der Herstellung von 5-Amino-1-(3,4,5-trichlorbenzyl)imidazol-4-carboxamid.

7. Ein Verfahren wie in Anspruch 1 oder 2 beansprucht, angewendet bei der Herstellung von 5-Amino-1-[4-(4-chlorphenylthio)-3-chlorbenzyl]imidazol-4-carboxamid.

8. Ein Verfahren wie in Anspruch 1 oder 2 beansprucht, angewendet bei der Herstellung von 5-Amino-1-[4-(4-chlorphenoxy)-3-chlorbenzyl)imidazol-4-carboxamid.

9. Eine Verbindung, erhalten nach einem wie in einem der Ansprüche 1 bis 8 beanspruchten Verfahren, zur Verwendung bei der Verabreichung an ein Tier, um Coccidiosis vorzubeugen oder zu behandeln.

10. Eine zur Coccidiosis-Vorbeugung und zur Coccidiosis-Behandlung nützliche Zusammensetzung, enthaltend einen inerten Träger und eine Verbindung, die nach einem wie in einem der Ansprüche 1 bis 8 beanspruchten Verfahren erhalten worden ist.

**Revendications pour les États contractants: BE CH DE FR GB IT LI LU NL SE**

1. Un composé répondant à la formule:

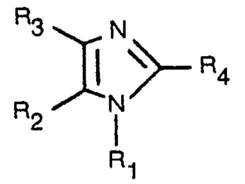

dans laquelle $R_1$ est (a) un phényle monosubstitué ou un phényl-alkyle en $C_{1-3}$ monosubstitué dont le substituant est un trifluorométhyle, un alcanoyle en $C_{2-3}$, un nitro, un carboxy, un alcoxycarbonyle, un acétamido, un alkylthio en $C_{1-3}$, un alkylsulfinyle en $C_{1-3}$, un alkylsulfonyle en $C_{1-3}$ ou

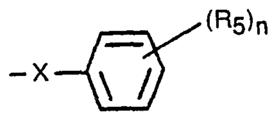

où n est de 0 à 5, $R_5$ est comme défini ci-dessous et X est O, S, SO, $SO_2$, $CH_2$, CO, CHOH, CHCN ou $C{=}NR_6$ où $R_6$ est un hydrogène, un alkyle en $C_{1-3}$, un hydroxy, un alcoxy en $C_{1-3}$, un amino, un alkylamino en $C_{1-3}$ ou un di(alkyl en $C_{1-3}$)amino; (b) un phényle ou un phénylalkyle en $C_{1-3}$ ayant 2 à 5 substituants $R_5$ où chaque $R_5$, indépendamment de l'autre ou des autres, est un halogéno, un cyano, un trifluorométhyle, un alcanoyle en $C_{2-3}$, un nitro, un alkyle en $C_{1-3}$, un alcoxy en $C_{1-3}$, un carboxy, un alcoxycarbonyle, un trifluorométhoxy, un acétamido, un alkylthio en $C_{1-3}$, un alkylsulfinyle en $C_{1-3}$, un alkylsulfonyle en $C_{1-3}$, un trichlorovinyle, un trifluorométhylthio, un trifluorométhylsulfinyle, un trifluorométhylsulfonyle ou

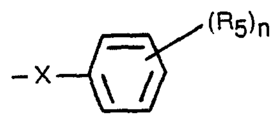

où X et n sont comme définis ci-dessus et $R_5$ est comme défini ci-dessus si ce n'est qu'il n'est pas

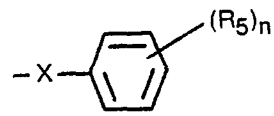

sous réserve que si un substituant est un halogéno, un alkyle en $C_{1-3}$ ou un alcoxy en $C_{1-3}$, ce substituant est en une position autre que la position ortho par rapport aux positions de fixation du phényle à l'imidazole ou à l'alkyle qui est lui-même fixé à l'imidazole; ou (c) un phénacyle, un pyridyle, un pyridylméthyle, un naphtyle, un naphtylméthyle, un quinolyle ou un quinolylméthyle;

$R_2$ est un amino, un alkylamino en $C_{1-3}$, un di(alkyl en $C_{1-3}$)amino, un acétamido, un acétimido, un uréido, un formamido, un formimido ou un guanidino;

$R_3$ est un carbamoyle, un cyano, un carbazoyle, un amidino ou un N-hydroxycarbamoyle; et

$R_4$ est un hydrogène, un alkyle en $C_{1-3}$, un hydroxy, un amino, un alkylamino en $C_{1-3}$, un di(alkyl en $C_{1-3}$)amino, un phényle, un cyano, un alcoxy, en $C_{1-3}$, un alcanoyloxy en $C_{2-3}$, un alkylthio en $C_{1-3}$, un alkylsulfinyle en $C_{1-3}$ ou un alkylsulfonyle en $C_{1-3}$.

2. Un composé comme revendiqué dans la revendication 1, dans lequel $R_1$ est un phényle monosubstitué ou un benzyle monosubstitué dont le substituant est un radical trifluorométhyle, phénoxy, benzoyle, phénylthio, phénylsulfinyle ou phénylsulfonyle ou un radical phénoxy, phénylthio, phénylsulfinyle, phénylsulfonyle, benzoyle ou phénylhydroxyméthyle, halogéno-substitué, méthyl-substitué ou trifluorométhyl-substitué; un radical phényle ou benzyle di- ou trisubstitué dont les substituants sont un radical halogéno, cyano, méthyle, trifluorométhyle, phénoxy, benzoyle, phénylthio, phénylsulfinyle, phénylsulfonyle ou phénoxy, phénylthio, phénylulfinyle, phénylsulfonyle, benzoyle ou phénylhydroxyméthyle halogeno-substitué, méthyl-substitué ou trifluorométhyl-substitué; sous réserve que si un substituant est un halogène, il est en une position autre que la position ortho par rapport à la position de fixation du phényle à l'imidazole ou au méthyle qui est lui-même fixé à l'imidazole; $R_2$ est un amino, un alkylamino en $C_{1-3}$ ou un di(alkyl en $C_{1-3}$)amino; $R_3$ est un carbamoyle; et $R_4$ est un hydrogène.

3. Un composé comme revendiqué dans la revendication 2, dans lequel $R_1$ est un radical phényle ou benzyle ayant 2 ou 3 substituants halogéno, cyano, méthyle, trifluorométhyle, halogénophénoxy, tolyloxy, trifluorométhylphénoxy, halogénophénylthio, tolylthio, trifluorométhylphénylthio, halogénophénylsulfinyle, tolylsulfinyle, trifluorométhylphénylsulfinyle, halogénophénylsulfonyle, tolylsulfonyle, trifluorométhylphénylsulfonyle, halogénobenzoyle, méthylbenzoyle, trifluorométhylbenzoyle, halogénophénylhydroxyméthyle, méthylphénylhydroxyméthyle et/ou trifluorométhylphénylhydroxyméthyle dans les positions méta et/ou para; $R_2$ est un amino; $R_3$ est un carbamoyle; et $R_4$ est un hydrogène.

4. 5-amino-1-(3,4,5-trichlorobenzyl)imidazole-4-carboxmide.

5. 5-amino-1-[4-(4-chlorophénylthio)-3-chlorobenzyl]imidazole-4-carboxamide.

6. 5-amino-1-[4-(4-chlorophénoxy)-3-chlorobenzyl]imidazole-4-carboxamide.

7. Un procédé pour la preparation d'un composé comme revendiqué dans la revendication 1, comprenant la réaction d'un composé répondant à la formule

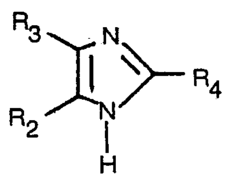

avec un halogénure $R_1$-substitué en présence d'une base pour produire le composé désiré, $R_1$, $R_2$, $R_3$ et $R_4$ étant comme défini dans la revendication 1.

8. Un procédé pour la préparation d'un composé comme revendiqué dans la revendication 1 et répondant à la formule

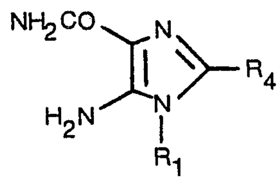

comprenant le traitement de l'aminocyanoacétamide avec une amine $R_1$-substituée en présence d'un composé de formule $(C_2H_5O)_3CR_4$ pour produire le composé désiré, $R_4$ étant un hydrogène, un alkyle en $C_{1-3}$ ou un phényle et $R_1$ étant comme défini dans la revendication 1.

9. Un composé comme revendiqué dans l'une quelconque des revendications 1 à 6 pour l'emploi dans l'administration à un animal pour la prévention ou le traitement de la coccidiose.

10. Une composition utile pour la prévention et le traitement de la coccidiose qui comprend un support inerte et un composé comme revendiqué dans l'une quelconque des revendications 1 à 6.

**Revendications pour l'État contractant: AT**

1. Un procédé pour la préparation d'un composé de formule:

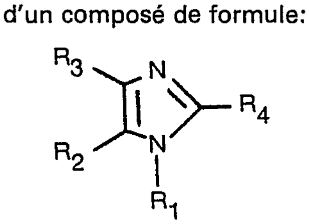

comprenant la réaction d'un composé répondant à la formule

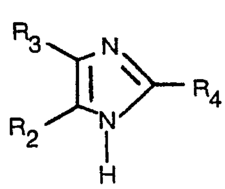

dans laquelle $R_1$ est (a) un phényle monosubstitué ou un phényl-alkyle en $C_{1-3}$ monosubstitué dont le substituant est un trifluorométhyle, un alcanoyle en $C_{2-3}$, un nitro, un carboxy, un alcoxycarbonyle, un acétamido, un alkylthio en $C_{1-3}$, un alkylsulfinyle en $C_{1-3}$, un alkylsulfonyle en $C_{1-3}$ ou

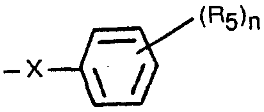

où n est de 0 à 5, $R_5$ est comme défini ci-dessous et X est O, S, SO, $SO_2$, $CH_2$, CO, CHOH, CHCN ou $C{=}NR_6$ où $R_6$ est un hydrogène, un alkyle en $C_{1-3}$, un hydroxy, un alcoxy en $C_{1-3}$, un amino, un alkylamino en $C_{1-3}$ ou un di(alkyl en $C_{1-3}$)amino; (b) un phényle ou un phénylalkyle en $C_{1-3}$ ayant 2 à 5 substituants $R_5$ où chaque $R_5$, indépendamment de l'autre ou des autres, est un halogéno, un cyano, un trifluorométhyle, un alcanoyle en $C_{2-3}$, un nitro, un alkyle en $C_{1-3}$, un alcoxy en $C_{1-3}$, un carboxy, un alcoxycarbonyle, un trifluorométhoxy, un acétamido, un alkylthio en $C_{1-3}$, un alkylsulfinyle en $C_{1-3}$, un alkylsulfonyle en $C_{1-3}$, un trichlorovinyle, un trifluorométhylthio, un trifluorométhylsulfinyle, un trifluorométhylsulfonyle ou

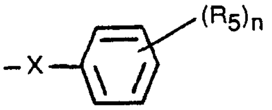

où X et n sont comme définis ci-dessus et $R_5$ est comme défini ci-dessus si ce n'est qu'il n'est pas

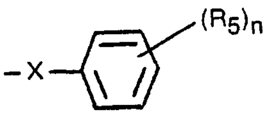

sous réserve que si un substituant est un halogéno, un alkyle en $C_{1-3}$ ou un alcoxy en $C_{1-3}$, ce substituant est en une position autre que la position ortho par rapport aux positions de fixation du phényle à l'imidazole ou à l'alkyle qui est lui-même fixé à l'imidazole; ou (c) un phénacyle, un pyridyle, un pyridylméthyle, un naphtyle, un naphtylméthyle, un quinolyle ou un quinolylméthyle;

$R_2$ est un amino, un alkylamino en $C_{1-3}$, un di(alkyl en $C_{1-3}$)amino, un acétamido, un acétimido, un uréido, un formamido, un formimido ou un guanidino;

$R_3$ est un carbamoyle, un cyano, un carbazoyle, un amidino ou un N-hydroxycarbamoyle; et

$R_4$ est un hydrogène, un alkyle en $C_{1-3}$, un hydroxy, un amino, un alkylamino en $C_{1-3}$, un di(alkyl en $C_{1-3}$)amino, un phényle, un cyano, un alcoxy en $C_{1-3}$, un alcanoyloxy en $C_{2-3}$, un alkylthio en $C_{1-3}$, un alkylsulfinyle en $C_{1-3}$ ou un alkylsulfonyle en $C_{1-3}$.

2. Un procédé pour la préparation d'un composé répondant à la formule

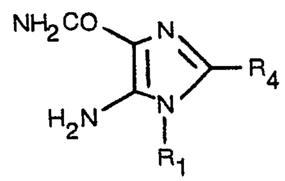

comprenant le traitement de l'aminocyanoacétamide avec une amine $R_1$-substituée en présence d'un composé de formule $(C_2H_5O)_3CR_4$ pour produire le composé désiré, $R_4$ étant un hydrogène, un alkyle en $C_{1-3}$ ou un phényle et $R_1$ étant comme défini dans la revendication 1.

3. Un procédé comme revendiqué dans la revendication 1 appliqué à la préparation d'un composé dans lequel $R_1$ est un phényle monosubstitué ou un benzyle monosubstitué dont le substituant est un radical trifluorométhyle, phénoxy, benzoyle, phénylthio, phénylsulfinyle ou phénylsulfonyle ou un radical phénoxy, phénylthio, phénylsulfinyle, phénylsulfonyle, benzoyle ou phénylhydroxyméthyle halogéno-substitué, méthyl-substitué ou trifluorométhyl-substitué; un radical phényle ou benzyle di- ou trisubstitué dont les substituants sont un radical halogéno, cyano, méthyle, trifluorométhyle, phénoxy, benzoyle, phénylthio, phénylsulfinyle, phénylsulfonyle ou phénoxy, phénylthio, phénylsulfinyle, phénylsulfonyle, benzoyle ou phénylhydroxyméthyle halogeno-substitué, méthyl-substitué ou trifluorométhyl-substitué; sous réserve que si un substituant est un halogène, il est en une position autre que la position ortho par rapport à la position de fixation du phényle à l'imidazole ou au méthyle qui est lui-même fixé à l'imidazole; $R_2$ est un amino, un alkylamino en $C_{1-3}$ ou un di(alkyl en $C_{1-3}$)amino; $R_3$ est un carbamoyle; et $R_4$ est un hydrogène.

4. Un procédé comme revendiqué dans la revendication 2 appliqué à la préparation d'un composé dans lequel $R_1$ est comme défini dans la revendication 3 et $R_4$ est un hydrogène.

5. Un procédé comme revendiqué dans la revendication 1 ou 2 appliqué à la préparation d'un composé dans lequel $R_1$ est un radical phényle ou benzyle ayant 2 ou 3 substituants halogéno, cyano, méthyle, trifluorométhyle, halogénophénoxy, tolyloxy, trifluorométhylphénoxy, halogénophénylthio, tolylthio,

trifluorométhylphénylthio, halogénophénylsulfinyle, tolylsulfinyle, trifluorométhylphénylsulfinyle, halogénophénylsulfonyle, tolylsulfonyle, trifluorométhylphénylsulfonyle, halogénobenzoyle, méthylbenzoyle, trifluorométhylbenzoyle, halogénophénylhydroxyméthyle, méthylphénylhydroxyméthyle et/ou trifluorométhylphénylhydroxyméthyle dans les positions méta et/ou para; et $R_4$ est un hydrogène.

6. Un procédé comme revendiqué dans la revendications 1 ou 2 applique à la préparation du 5-amino-1-(3,4,5-trichlorobenzyl)imidazole-4-carboxamide.

7. Un procédé comme revendiqué dans la revendications 1 ou 2 appliqué à la préparation du 5-amino-1-[4-(4-chlorophénylthio)-3-chlorobenzyl]imidazole-4-carboxamide.

8. Un procédé comme revendiqué dans la revendication 1 ou 2 appliqué à la préparation de 5-amino-1-[4-(4-chlorophénoxy)-3-chlorobenzyl]imidazole-4-carboxamide.

9. Un composé obtenu par un procédé comme revendiqué dans l'une quelconque des revendications 1 à 8 pour l'utiliser dans l'administration à un animal en vue de prévenir ou de traiter la coccidiose.

10. Une composition utile pour la prévention et le traitement de la coccidiose, qui comprend un véhicule inerte et un composé obtenu par un procédé comme revendiqué dans l'une quelconque des revendications 1 à 8.